# EUROPEAN PATENT SPECIFICATION

(11) **EP 2 929 855 B1**
(45) Date of publication and mention of the grant of the patent: **07.04.2021**
(21) Application number: 12889074.6
(22) Date of filing: 28.11.2012
(51) Int. Cl.: A61C 9/00, A61C 19/00, A61B 1/00, A61B 1/24, A61B 1/247, A61C 13/00

(54) **DENTAL SCANNING DEVICE**
DENTALE ABTASTVORRICHTUNG
DISPOSITIF DE SCANNAGE DENTAIRE

(43) Date of publication of application: 14.10.2015
(73) Proprietor: APOLLO ORAL SCANNER, LLC, Miami FL 33130 (US)
(72) Inventor: FERNÁNDEZ PULIDO, Alfonso, E-28224 Pozuelo de Alarcón (Madrid) (ES); DE PABLOS GARCÍA, David, E-28224 Pozuelo de Alarcón (Madrid) (ES)
(74) Representative: Schulz Junghans Patentanwälte PartGmbB
(86) International application number: PCT/ES2012/070834
(87) International publication number: WO 2014/083211

(56) References cited:
- WO-A1-91/03980
- US-A- 3 382 781
- US-A- 4 575 805
- US-A1- 2002 055 082
- US-A1- 2005 019 732
- US-A1- 2006 154 198

## Description

The present invention relates to a dental scanning device to be applied both in the field of stomatology in general, and in dental prosthetics manufacturing, and to a method for performing a dental scan by means of said dental scanning device.

More specifically, the invention relates to an intraoral dental scanning device making it possible to obtain a 3D image of the teeth and gums on a corresponding viewing device. No manual handling whatsoever by an operator is necessary, as the present device is fully automated; it is inserted into a patient's oral cavity, where the patient holds the device by exerting a force or pressure when biting it, the patient himself thereby constituting the fixed-reference system for the scan.

3D images of the teeth and gums for diagnostic and therapeutic purposes have traditionally been obtained using replicas or models from alginate-impressed molds. Such replicas make it possible to obtain a negative image of the gums and teeth, which must subsequently be cast and scanned. However, these conventional techniques pose problems in terms of both patient discomfort, and their lack of reliability and accuracy, making the process slow and costly.

In the state of the art there are several known devices that try to solve the problems posed by such conventional techniques; e.g. panoramic dental X rays or computerized dental tomographies.

In this respect, document EP 0825837, "Modular intra-oral imaging system video camera", for instance, essentially provides a hand-held video camera for capturing images from inside a patient's mouth, wherein the camera comprises a housing inside which there is a handle portion and a viewing portion, as well as a sensor device optically aligned along a longitudinal axis through said housing, and whose purpose is converting to data images taken by the camera.

Document US 2006154198, "3D dental scanner relates to a method and to a system for imaging a dental structure from the inside of the oral cavity, through the motion of at least one image capturer set on an fixed-reference-system coupled arm, external to the mouth, to generate a 3D model of the structure, based on the images captured.

Document ES2 383 220, "Sensor de imagen dental intraoral y sistema radiológico que utiliza dicho sensor" [Intraoral dental imaging sensor and X ray system using said sensor] describes an intraoral dental radiological system equipped with a mouth-insertable X ray imaging sensor made up of an image-detection matrix to provide electronic signals, wherein the system comprises a light source to receive the signals from the matrix, which emits binary light impulses corresponding to a digital image to be transmitted, and a light receiver placed at a certain distance from the patient, which can detect a light modulation triggered by the light source, and can transmit to an image-processing device a signal corresponding to this modulation.

Document ES2324658 (T3), "Sistema digitalizador por laser para aplicaciones dentales" [Laser digitizing system for dental applications] relates to a laser digitizer that comprises a light source with collimation optics configured to generate a collimated light beam; a scanner optically coupled with the light source and configured to scan the collimated beam towards the object to be optically represented in a predetermined pattern; an imaging instrument with an optical axis at an angle θ to the scanner, configured to detect a reflection of the pattern from the object, and generate the data representing the surface of the object, based on the reflection of the pattern; and a processor coupled to the scanner, and the imaging system, configured to render a data-based 3D image of the object; characterized in that the scanner is configured to scan the collimated beam all along at least two axes in the predetermined pattern, the pattern comprising a set of curvilinear segments.

Lastly, document WO 91/03980 A1 discloses a dental scanning device having a bite body and a scanner body housing a mobile scan head arranged to perform a sweeping motion in two directions.

These devices and systems from the state of the art have several drawbacks. First, they are essentially devices where, in some cases, a technician must manually operate them with a toothbrush-like motion, with the inaccuracies that this sort of manual operation might entail. Likewise, they are based on photographs taken by the various devices, wherein a software program usually interprets and interpolates these photographs to produce a final 3D image. Thus, besides the time needed to obtain the final image, these known systems depend on the operator's skill at handling the camera and inserting it in hard-to-reach parts of the mouth. Other known devices do not depend so much on manual handling by the operator, but have an external fixed coordinate system that is independent of the patient; therefore, the final image is exclusively based on images captured with no extra projection. Thus, they are not very accurate devices, as the reference system changes with the slightest movement of the patient.

Therefore, the object of this invention is a dental scanning device without the flaws of the previously known systems from the state of the art, and which makes it possible to obtain 3D images with respect to a fixed reference system that is the patient's own mouth. This enables a high level of accuracy in forming images, as the present device moves along with the patient; i.e. the reference of the position of the teeth, for instance, with respect to the device of the invention, is preserved at all moments, and remains fixed throughout the scanning process.

To do so, the dental scanning device of the invention as defined in claim 1 is made up of two bodies; one of them houses the scanner as such, as well as a mobile scan head, and the other one is to be held by the patient biting, and is to correspondingly house the mobile scan head. Upon inserting the portion of the scan head housed in the bite body -and therefore held by the patient's mouth-the scan head performs a sweeping motion in at least two directions: one, following the longitudinal axis of the scan head, and the other one perpendicular to it, in both senses of the direction, such that scanning is the result of a combination of both movements: lateral and depth-wise, with respect to the dental arcade. This is why the body to be held by the patient's mouth is made of an easy-to-disinfect/sterilize material.

According to the invention, the aforementioned bodies can be coupled to one another, and may be disassembled and joined again; in this case, either the patient's piece intended to be held by the patient's mouth is made of an easy-to-disinfect/sterilize material, or constitutes an element that is disposable after use. In this embodiment according to the invention, the scanner body (1) and the bite body (2) are easily coupled to one another through corresponding means arranged on their the coupling ends.

What follows is a description of the invention, based on one of the preferred embodiments thereof, and with reference to the attached figures, wherein:
- Figure 1: is a plan view of the scanning device according to one embodiment of the invention.
- Figure 2: is a side view of the scanning device in figure 1, showing how it is inserted into the patient's mouth.
- Figure 3: is a diagram showing the scan directions of the device in figure 1.

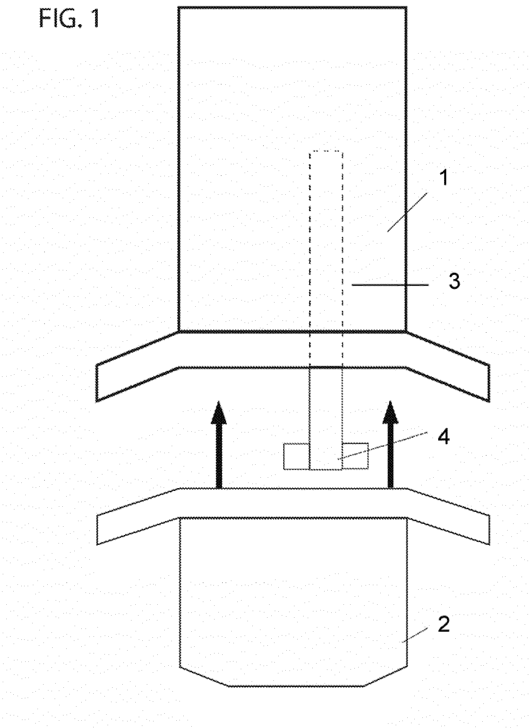

As shown in figure 1, the dental scanning device of the invention is made up of two bodies. The first one, the scanner body (1), houses a mobile scan head (3) made up of a mobile longitudinal element, ending in the scan head (4) as such. This mobile element (3, 4) is partially kept inside the scanner body (1). On the other hand, the scan head (4) projects from the body (1) for the purpose of being housed in a second body or bite body (2) by way of a case. The bite body (2) therefore constitutes a case to house the scan head (4).

If the abovementioned bodies can be coupled, and may be separated and joined again, the foregoing is equally applicable when both bodies (1 & 2) have been previously coupled to one another.

As indicated, the bite body (2) is inserted into the patient's mouth and is held in place when the patient bites it, such that the scan head (4) is inserted into the mouth to scan the corresponding dental arcade.

Likewise, the shape of the bite body (2) adapts to the mouth to make it easier to hold. This bite body (2) essentially constitutes a protective case for the scan head (4), and can be bitten by the user. This is why the bite body (2) is made of a suitable transparent material.

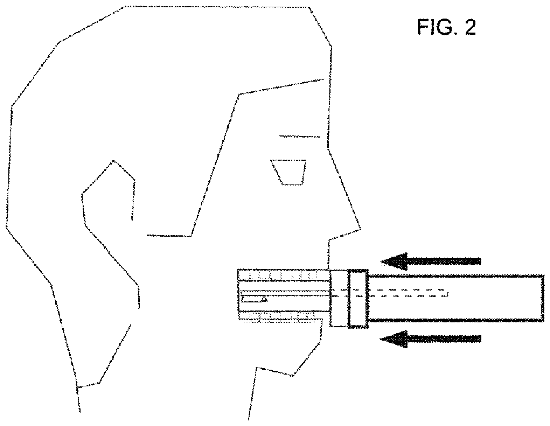

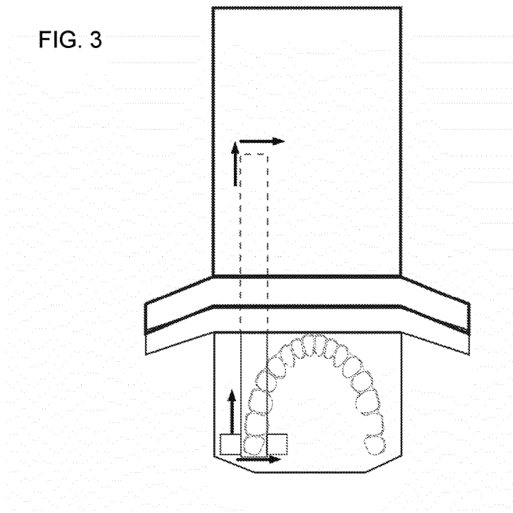

Once the part of the scan head (4) that is housed in the bite body (2) has been inserted, and is thereby secured in the patient's mouth, as shown in figure 2, the scan head performs a sweeping motion in at least two directions, one following the longitudinal axis of the longitudinal element (3), and the other one perpendicular to it, in both senses of the direction, such that scanning is the result of a combination of both movements: lateral and depth-wise, with respect to the dental arcade, as shown in figure 3.

For intraoral scanning, the scan head (4) of the device of the invention includes detection sensors, laser sensors or similar, as well as cameras to capture images derived from the tooth-by-tooth sweep of the dental arcade and the gums. These are obtained automatically, and, as a result of the design with a fixed and constant reference system, can be used to generate an accurate 3D image.

## Claims

1. A dental scanning device made up of two bodies (1, 2) wherein the first body, also termed scanner body (1) houses a mobile scan head (3) consisting of a mobile longitudinal element ending in the scan head (4) as such, and wherein the second body, also termed bite body (2) constitutes a case to house the scan head (4), as well as the element for the patient to secure and place the device by biting it,
wherein the scan head (4) is adapted to perform a sweeping motion in at least two directions: one following the longitudinal axis of the longitudinal element (3), and the other one perpendicular to it, in both senses of the direction, such that scanning is the result of a combination of both movements: lateral and depth-wise, with respect to the dental arcade, **characterised in that** the bodies (1, 2) are separable and couple to one another through corresponding means arranged on respective coupling ends of the first body (1) and of the second body (2).

2. The dental scanning device according to claim 1, **characterized in that** the mobile scan head (3,4) is partially kept inside the scanner body (1).

3. The dental scanning device according to claim 1, **characterized in that** the scan head (4) projects from the body (1) for the purpose of being housed in the second body or bite body (2) by way of a case.

4. The dental scanning device according to claim 1, **characterized in that** the bite body (2) is manufactured with a transparent material.

5. The dental scanning device according to claim 1, **characterized in that** the bite body (2) is disposable after use.

6. The dental scanning device according to one of the preceding claims, **characterized in that** the sweeping motion is done with respect to a fixed reference system that is the patient's own mouth, wherein the second body or bite body (2) is held by the patient's mouth by exerting pressure when biting it.

7. A method for performing a dental scan by means of the dental scanning device according to one of the claims 1 to 6, comprising the steps of:
- inserting the bite body (2) of the dental scanning device into the patient's mouth,
- holding the bite body (2) in place, when the patient bites the bite body (2),
- inserting the scan head (4) of the dental scanning device into the patient's mouth, particularly into the bite body (2),
- scanning a corresponding dental arcade, wherein a sweeping motion is performed in at least two directions: one following the longitudinal axis of the longitudinal element, and the other one perpendicular to it, in both senses of the direction, such that scanning is the result of a combination of both movements: lateral and depth-wise, with respect to the dental arcade,
wherein the scanner body (1) and the bite body (2) are coupled to each other.

8. The method according to claim 7, wherein the sweeping motion is done with respect to a fixed reference system that is the patient's own mouth, and wherein the second body or bite body (2) is held by the patient's mouth by exerting pressure when biting it.

## Patentansprüche

1. Ein Zahnabtast-Gerät, zusammengesetzt aus zwei Körpern (1, 2), wobei der erste Körper, auch Abtastkörper (1) genannt, einen mobilen Abtastkopf (3) beherbergt, bestehend aus einer mobilen Längselement-Endung im Abtastkopf (4) als solchem, und wobei der zweite Körper, auch Beißkörper (2) genannt, ein Gehäuse zur Aufnahme des Abtastkopfes (4) bildet, sowie das Element für den Patienten, um das Gerät durch Beißen zu sichern und zu platzieren, wobei der Abtastkopf (4) angepasst ist, um eine Schwenkbewegung in mindestens zwei Richtungen durchzuführen: eine, die der Längsachse des Längselements (3) folgt, und die andere senkrecht dazu, in beiden Richtungen, so dass das Abtasten das Ergebnis einer Kombination beider Bewegungen ist: seitwärts und in die Tiefe, in Bezug auf den Zahnbogen, **dadurch gekennzeichnet, dass** die Körper (1,2) trennbar sind und
durch entsprechende Mittel, die an jeweiligen Kopplungsenden des ersten Körpers (1) und des zweiten Körpers (2) angeordnet sind, miteinander gekoppelt werden.

2. Das Zahnabtast-Gerät gemäß Anspruch 1, **dadurch gekennzeichnet, dass** der mobile Abtastkopf (3,4) teilweise innerhalb des Abtastkörpers (1) gehalten wird.

3. Das Zahnabtast-Gerät gemäß Anspruch 1, **dadurch gekennzeichnet, dass** der mobile Abtastkopf (4) aus dem Körper (1) ragt, um mittels eines Gehäuses in dem zweiten Körper oder Beißkörper (2) aufgenommen ist.

4. Das Zahnabtast-Gerät gemäß Anspruch 1, **dadurch gekennzeichnet, dass** der Beißkörper (2) mit einem transparenten Material hergestellt ist.

5. Das Zahnabtast-Gerät gemäß Anspruch 1, **dadurch gekennzeichnet, dass** der Beißkörper (2) nach Gebrauch entsorgbar ist.

6. Das Zahnabtast-Gerät gemäß einem der vorhergehenden Ansprüche, **dadurch gekennzeichnet, dass** die Schwenkbewegung in Bezug auf ein festes Referenzsystem erfolgt, welches der eigene Mund des Patienten ist, wobei der zweite Körper oder Beißkörper (2) vom Mund des Patienten durch Ausüben von Druck beim Beißen gehalten wird.

7. Das Verfahren zur Durchführung eines Zahnabtastvorgangs mittels des Zahnabtast-Geräts gemäß einem der Ansprüche 1 bis 6, umfassend die Schritte:
- Einführen des Beißkörpers (2) des Zahnabtast-Geräts in den Mund des Patienten,
- Halten des Beißkörpers (2) in Position, wenn der Patient auf den Beißkörper (2) beißt,
- Einführen des Abtastkopfes (4) des Zahnabtast-Geräts in den Mund des Patienten, insbesondere in den Beißkörper (2),
- Abtasten eines entsprechenden Zahnbogens, wobei eine Schwenkbewegung in mindestens zwei Richtungen ausgeführt wird: eine der Längsachse des Längselements folgend, und die andere senkrecht dazu, in beiden Richtungen, so dass das Abtasten das Ergebnis einer Kombination beider Bewegungen ist: seitlich und in die Tiefe, in Bezug auf den Zahnbogen, wobei der Abtastkörper (1) und der Beißkörper (2) miteinander gekoppelt sind.

8. Das Verfahren nach Anspruch 7, wobei die Schwenkbewegung in Bezug auf ein festes Bezugssystem erfolgt, welches der eigene Mund des Patienten ist, und wobei der zweite Körper oder Beißkörper (2) durch Ausüben von Druck beim Beißen vom Mund des Patienten gehalten wird.

## Revendications

1. Dispositif de scannage dentaire constitué de deux corps (1, 2),
dans lequel le premier corps, également appelé corps de scanner (1) héberge une tête de balayage mobile (3) constituée d'un élément longitudinal mobile se terminant dans la tête de balayage (4) tel quel, et dans lequel le second corps, également appelé corps à mordre (2) constitue un boîtier pour héberger la tête de balayage (4), ainsi que l'élément pour que le patient sécurise et place le dispositif en le mordant, dans lequel la tête de balayage (4) est adaptée à réaliser un mouvement de balayage dans au moins deux directions : une suivant l'axe longitudinal de l'élément longitudinal (3) et l'autre perpendiculaire à lui, dans les deux sens de la direction, de sorte qu'un balayage est le résultat d'une combinaison des deux mouvements :
latéral et en profondeur, par rapport à l'arcade dentaire, **caractérisé en ce que** les corps (1, 2) sont séparables et couplés l'un à l'autre par des moyens correspondants agencés sur des extrémités de couplage respectives du premier corps (1) et du second corps (2).

2. Dispositif de scannage dentaire selon la revendication 1, **caractérisé en ce que** la tête de balayage mobile (3, 4) est partiellement conservée à l'intérieur du corps de scanner (1).

3. Dispositif de scannage dentaire selon la revendication 1, **caractérisé en ce que** la tête de balayage (4) fait saillie depuis le corps (1) dans le but d'être hébergée dans le second corps ou corps à mordre (2) au moyen d'un boîtier.

4. Dispositif de scannage dentaire selon la revendication 1, **caractérisé en ce que** le corps à mordre (2) est fabriqué en un matériau transparent.

5. Dispositif de scannage dentaire selon la revendication 1, **caractérisé en ce que** le corps à mordre (2) est jetable après usage.

6. Dispositif de scannage dentaire selon une des revendications précédentes, **caractérisé en ce que** le mouvement de balayage est réalisé par rapport à un système de référence fixe qui est la propre bouche du patient, dans lequel le second corps ou corps à mordre (2) est maintenu par la bouche du patient en exerçant une pression lors de sa morsure.

7. Procédé de réalisation d'un examen dentaire par balayage au moyen du dispositif de scannage dentaire selon une des revendications 1 à 6, comprenant les étapes consistant à :
- insérer le corps à mordre (2) du dispositif de scannage dentaire dans la bouche du patient,
- maintenir le corps à mordre (2) en place lorsque le patient mord le corps à mordre (2),
- insérer la tête de balayage (4) du dispositif de scannage dentaire dans la bouche du patient, notamment dans le corps à mordre (2),
- balayer une arcade dentaire correspondante, dans lequel un mouvement de balayage est réalisé dans au moins deux directions : une suivant l'axe longitudinal de l'élément longitudinal et l'autre perpendiculaire à lui, dans les deux sens de la direction, de sorte qu'un balayage est le résultat d'une combinaison des deux mouvements : latéral et en profondeur, par rapport à l'arcade dentaire,
dans lequel le corps de scanner (1) et le corps à mordre (2) sont couplés l'un à l'autre.

8. Procédé selon la revendication 7, dans lequel le mouvement de balayage est réalisé par rapport à un système de référence fixe qui est la propre bouche du patient, et dans lequel le second corps ou corps à mordre (2) est maintenu par la bouche du patient en exerçant une pression lors de sa morsure.
